**Europäisches Patentamt**

(19) **European Patent Office**

**Office européen des brevets**

(11) Publication number : **0 417 193 B1**

(12) # EUROPEAN PATENT SPECIFICATION

(45) Date of publication of patent specification :
04.08.93 Bulletin 93/31

(51) Int. Cl.⁵ : **A61K 39/395, A61K 47/00**

(21) Application number : **89906925.6**

(22) Date of filing : **24.05.89**

(86) International application number :
**PCT/US89/02252**

(87) International publication number :
**WO 89/11297 30.11.89 Gazette 89/28**

(54) **FREEZE-DRIED FORMULATION FOR ANTIBODY PRODUCTS.**

(30) Priority : **27.05.88 US 199931**

(43) Date of publication of application :
**20.03.91 Bulletin 91/12**

(45) Publication of the grant of the patent :
**04.08.93 Bulletin 93/31**

(84) Designated Contracting States :
**AT BE CH DE FR GB IT LI LU NL SE**

(56) References cited :
EP-A- 73 371
EP-A- 0 124 018
CH-A- 0 645 537
US-A- 4 186 192

(73) Proprietor : **CENTOCOR, INC.**
**244 Great Valley Parkway**
**Malvern, PA 19355 (US)**

(72) Inventor : **PHILLIPS, Christopher, P.**
**P.O. Box 65**
**Brandamore, PA 19316 (US)**
Inventor : **MATTIS, Jeffrey, A.**
**1220 Upton Circle**
**West Chester, PA 19380 (US)**

(74) Representative : **Harvey, David Gareth et al**
**Graham Watt & Co. Riverhead**
**Sevenoaks Kent TN13 2BN (GB)**

## Description

Background of the Invention

A major challenge that exists in the field of new protein drugs is in the formulation of protein solution products that maintain both protein solubility and biological activity. It is well known that purified monoclonal antibodies, for example, tend to precipitate from the solution when subjected to physical or chemical stress, or over time. Many protein preparations are unstable in solution, and the instability is manifested in the formation of insoluble particles in the solution. The formation of these particles is often increased by physical stress, such as storage for prolonged periods and shipping; and by chemical stress such as dilution or mixing with an additive. Such particulates are not acceptable in terms of visual appearance of the product or USP specifications, and cause much difficulty in the development of suitable liquid formulations of monoclonal antibodies intended for intravenous injection.

The precipitation of proteins from aqueous solutions is a well known phenomenon. Immunoglobulins, in particular, possess characteristics that tend to generate particles in solution, so that an immunoglobulin solution generally must be filtered before used for intravenous injection.

Conditions which lead to increased precipitation of immunoglobulins include long term storage, filling vials with automated equipment and shipping.

Various methods for stabilizing protein preparations have been used, including increasing the concentrations of protein in solution or adding an additional protein, such as human serum albumin. However, such preparations may not be acceptable for therapeutic purposes.

Various carbohydrates are known to stabilize and/or enhance the stability of certain biologically active protein preparations. See, EPO 124 018, Landaburu et al.; (1984) and CH 645,537, Uemura et al., (1981). For example, U.S. Patent 4,186,192 to Lundblad et al., discloses the use of maltose to increase the stability of an immune serum globulin preparation for intramuscular or intravenous administration. T. Arakawa and S.N. Timasheff describe the stabilization of protein structure by lactose and glucose in Biochemistry, 21:6536-6544 (1982). Gazzei et al. describe the addition of saccharose to a formulation of immunoglobulins in European Patent Application 221,505.

Maltose has been added to protein solutions to enhance stability of the proteins in solutions and to impart isotonicity to a solution. Maltose has many advantages, including availability in pure form and stability in aqueous solutions. Preparations containing maltose can be autoclaved without browning of the solution. Maltose in small quantities is practically physiologically inert. When administered parenterally, it is gradually converted to glucose by the enzyme maltase; however, the conversion to glucose is gradual and frequently undetectable when serum glucose is measured. Therefore, there is no detectable increase in circulating insulin levels. In U.S. Patent 4,449,073, Tenold discloses the use of maltose in a preparation of immune serum golubin to import physiologically acceptable isotonicity to the preparation. Tenold specifies 10% weight to volume maltose for this purpose. Lundblad et al., report in U.S. Patent 4,186,192 that the stability of a solution of immune serum globulin is increased by adding maltose in a concentration between 5 and 18% by weight. Fernandes et al. describes a preparation of intravenous gamma globulin stabilized with maltose to minimize precipitation and improve in vitro shelf stability in Vox Sang, 39:101-112 (1980).

One approach used to circumvent this problem is the development of lyophilized drug forms. Lyophilization is the freeze-drying of biological material. Generally, the material is first frozen and then placed in a high vacuum so that the water (in the form of ice) sublimes into the vacuum and the non-water components are left behind in an undamaged state. The resulting product is a cake-like substance which can then be stored for long periods of time without degradation. The powder is reconstituted at the time of use to a liquid product by the addition of water, saline or other diluent. The reconstituted solution is then ready to be administered to the subject.

Summary of the Invention

This invention relates to a lyophilized composition containing a monoclonal antibody, a buffer and maltose, intended for parenteral administration. The invention further relates to a method for preparing the lyophilized product. The lyophilized composition of the invention preserves the biological activity of the monoclonal antibody, and minimizes formation of particulates caused by precipitation or aggregation of the monoclonal antibody when the freeze-dried product is reconstituted. The reconstituted solutions are particle-free, show no loss of biological activity and can be administered without prior filtration.

Brief Description of the Drawings

Figure 1 shows the gel filtration HPLC results comparing lyophilized and liquid samples of IgG exposed to a range of stress temperatures for 95 days.

Figure 2 shows the results of an immunoreactivity assay comparing binding ability of both lyophilized and 10% maltose liquid samples of IgG which have been exposed to a range of stress temperatures for 95 days.

Figure 3 shows the HPLC gel filtration results of lyophilized 2% maltose samples exposed to a range of stress temperatures for 52 days.

Figure 4 shows the results of an immunoreactivity assay for lyophilized samples of 2% maltose exposed to a range of stress temperatures for 52 days.

Detailed Description of the Invention

The formulation comprises a lyophilized composition containing a low pH buffer, a carbohydrate component and an antibody or antibody fragment. The liquid composition is prepared, then lyophilized to form a cake-like product. The lyophilized product can be stored for prolonged periods of time, and at elevated temperatures, without loss of biological activity, and is readily reconstituted into a particle-free solution by the addition of water, saline or other diluent.

Lyophilization is a freeze drying process which is often used in the preparation of pharmaceutical products to preserve their biological activity. The process generally involves drying a previously frozen sample in a vacuum to remove the ice, leaving the non-water components intact, in the form of a powdery or cake-like substance. The dried product is then be reconstituted at the time of use by the addition of water, saline solution, buffer or other appropriate liquid to produce a solution of the antibody. The advantage of lyophilization is that the biological activity of the antibody is preserved, and solution stress caused by exposure to varying temperatures, shear forces and shipping is avoided. The lyophilized product is readily reconstituted and is particle free, so it can be administered intravenously without prior filtration.

One embodiment of the present formulation is a solution containing a buffer, maltose and a murine monoclonal antibody or antibody fragments, such as immunoglobulin G (IgG), which is lyophilized to form a cake-like product.

All subclasses of immunoglobulin G can be used, e.g. immunoglobulin $G_1$, $G_2$, $G_{2a}$, $G_{2b}$ and $G_3$. The antibody or antibody fragment can be derivatized so that it binds with a radiometal, such as Technetium-99m or Indium-111. The antibody can be derivatized either by changing its native state so that a radiometal can be bound directly to the protein, or with a chelating agent such as diethylenetriaminepentaacetic acid (DTPA). The derivatized antibody can be used as a radio pharmaceutical due to the derivatized antibody's ability to bind a radioactive heavy metal (e.g. Indium-111 or Technetium-99m) to the antibody protein, forming a protein-radiometal complex, or, in the case of a chelating agent, a protein-chelate-radiometal complex. The radiolabeled antibody can then be used in immunoscintigraphy, for example, for in vivo diagnostic imaging of tumors or disease sites. IgG labeled with a radiation source, such as Tc-99m, can be administered to a subject. The labeled antibody localizes at the site defined by anti-IgG, and the site can then be scanned with a gamma camera, and an image obtained.

A preferred embodiment of the invention is a formulation containing a buffer having a low pH (between 3 to 6), maltose and IgG. Preferred buffers include sodium acetate, phosphate and citrate buffers. A more preferred embodiment is a formulation containing 5-10 mM sodium acetate, having a pH between 3-6, 2-10% maltose and 1 to 25 mg/ml IgG.

The liquid formulation can be lyophilized using appropriate drying parameters. The following drying parameters are preferred: a primary drying phase temperature of -40°C to -42°C and pressure between 6.67 to 10.7 Pa (0.05 millimeters of mercury (mmHg) to 0.08 mmHg (50 mTorr to 80 mTorr)): and a secondary drying phase at ambient temperature, and pressure between 6.67 to 10.7 Pa (0.05 mmHg to 0.08 mmHg (50 mTorr to 80 mTorr)).

Maltose, which is used to stabilize the antibody solutions, is described in detail in, for example, the Merck Index, 10th edition, Merck and Co., Inc. Rohway, NJ (1983). Maltose is a dissaccharide (4-O-D-glucopyranosyl-D-glucopyranose) having the general formula $C_{12}H_{22}O_{11}$, which has been established as useful for stabilizing immunoglobulin solutions. (See U.S. Patent 4,499,073 to Tenold and U.S. Patent 4,186,192 to Lundblad, et al.). Maltose is not metabolized by humans when administered intravenously, and is excreted as maltose with no apparent elevation in blood glucose levels or release of insulin.

The lyophilized product is redissolved at the time of use in a diluent (e.g., sterile water or saline) and yields a particle-free solution. Reconstitution is complete in about two minutes. The reconstituted antibody solution is particle-free even after prolonged storage of the lyophilized cake at 37°C. The reconstituted solution is then

3

administered parenterally, preferably intravenously, to the subject.

The invention is further illustrated by the following exemplification:

Exemplification

Example 1. Preparation of a Stabilized Lyophilized Formulation of Immunoglobulin G.

Desalting of IgG from 0.9% Saline Into a solution of 10mM Sodium Acetate (pH 4.5) containing 10% Maltose

A 3.3 L Sephadex® G-25 medium column (Pharmacia) packed in 0.1 M sodium hydroxide was equilibrated with a solution of 10 mM sodium acetate (pH 4.5) and 10% w/v maltose before applying the protein. 500 ml of IgG (Centocor, Inc., Malvern, PA) at a concentration of 11.54 mg/ml (5.693g total) was applied to the column at a flow rate of 550 ml/hr. The protein peak was collected and concentrated to 10 mg/ml via stirred cell with YM30-membrane (Amicon).

The entire lot was split in half, and one part was maintained in the liquid state, and the other dedicated for freeze-drying. Each part consisted of 25 vials that were subjected to temperature stress testing upon the completion of the lyophilization process.

Lyophilization of IgG Formulation

Twenty milliliter vials (West Co.) were filled with 10 ml of the formulation and placed in the freeze dryer (FTS). Since freeze-drying was not under sterile conditions, 100 ul of 10% sodium azide was added to the lyophilized samples as a baceteriostatic agent.

Placebo vials, filled with the identical buffer and maltose but lacking antibody, were also placed in the lyophilizer. The degree of supercooling of the formulation was -6°C and formed a uniform matrix. The product was initially frozen to -45°C. Ater freezing to this temperature, the shelf surface temperature was adjusted to between -40 to -42°C, with the pressure reduced to 6.67 to 10.7 Pa (0.05 mmHg (50 mTorr)) to initiate the primary drying phase.

Once the primary drying phase was completed, the shelf surface temperature was raised to +20°C to initiate the secondary drying phase. The composition of the gases in the chamber were monitored via residual gas mass spectrum until nitrogen had replaced water vapor as the species in the chamber. The secondary drying phase required about 8 hours.

After reaching this point, the chamber was back-filled with dry nitrogen and the vials were stoppered.

The procedure yielded 25 vials of lyophilized product at 10 ml/vial, 10.0 mg/ml.

Temperature Stress Study

Representative vials from both the liquid and freeze-dried formulations were exposed to temperatures of 4, 22, 37 and 50°C.

After 95 days under such conditions, one vial from each formulation at each temperature was removed for analysis. The lyophilized product was reconstituted with 10 ml of water. The reconstitution times are shown in Table 1 below.

## Table 1

### Reconstitution Times (Lyophilized Product)

| Sample | Reconstitution Time (Minutes) |
|---|---|
| $t_o$ sample | 1 |

---

**95 day samples**

| | |
|---|---|
| 4°C | 2 |
| 22°C | 2 |
| 37°C | 2.5 |
| 50°C | 2 |

Each vial was then visually examined for particulates. The vials were examined by holding the vial directly in front of a black background and lighting the vial from below. Visual comparison of reconstituted lyophilized product to liquid samples demonstrated the superiority of the lyophilized product. The liquid samples all contained particulation of some degree, whereas the reconstituted samples were clear and free of particulates. The liquid samples also showed increased yellowing as the temperature increased. The results of the visual examination are shown in Table 2.

## Table 2

## Visual Examination

| T°C | Formulation | Observation |
|-----|-------------|-------------|
| 4 | liquid | small flakes |
| 22 | liquid | small flakes and fine precipitation |
| 37 | liquid | fine particulates, slightly yellow liquid |
| 50 | liquid | moderate particulation, yellow liquid |
| 4 | lyophilized | clear |
| 22 | lyophilized | clear |
| 37 | lyophilized | clear |
| 50 | lyophilized | clear |

Liquidborne Particle Analysis

The vials were then analyzed in a liquidborne particle analyzer (Climet). The samples were analyzed for particles ranging in size from 5 to 50 $\mu$m. Subvisible liquidborn particle analysis showed some superiority of the reconstituted lyophilized samples over the liquid samples. The results are shown in Table 3.

6

## Table 3

### Subvisible Liquidborne Particle Analysis
#### (particles per milliliter)

| T°C Formulation | Differential | | | | | | Total | | | | | |
|---|---|---|---|---|---|---|---|---|---|---|---|---|
| | 5µ | 10µ | 20µ | 25µ | 30µ | 50µ | 5µ | 10µ | 20µ | 25µ | 30µ | 50µ |
| 4 Liquid | 188 | 92 | 18 | 12 | 50 | 172 | 532 | 344 | 242 | 234 | 222 | 172 |
| 22 Liquid | 182 | 50 | 6 | 2 | 6 | 0 | 246 | 64 | 14 | 8 | 6 | 0 |
| 37 Liquid | 282 | 102 | 12 | 4 | 10 | 2 | 412 | 130 | 28 | 16 | 12 | 2 |
| 50 Liquid | 204 | 92 | 14 | 6 | 10 | 4 | 330 | 126 | 34 | 20 | 14 | 4 |
| 4 Lyophilized | 230 | 64 | 4 | 2 | 0 | 0 | 300 | 70 | 6 | 2 | 0 | 0 |
| 22 Lyophilized | 236 | 42 | 4 | 2 | 2 | 0 | 256 | 50 | 8 | 4 | 2 | 0 |
| 37 Lyophilized | 194 | 28 | 4 | 2 | 2 | 0 | 230 | 36 | 8 | 4 | 2 | 0 |
| 50 Lyophilized | 550 | 82 | 14 | 10 | 8 | 0 | 664 | 114 | 32 | 18 | 8 | 0 |

### HPLC Gel Filtration Chromatography

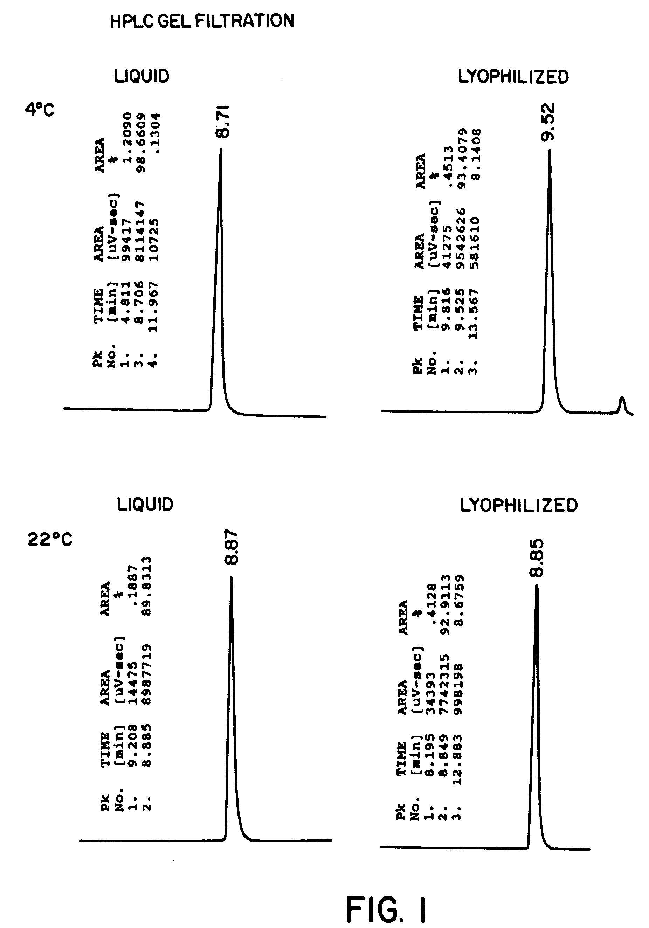

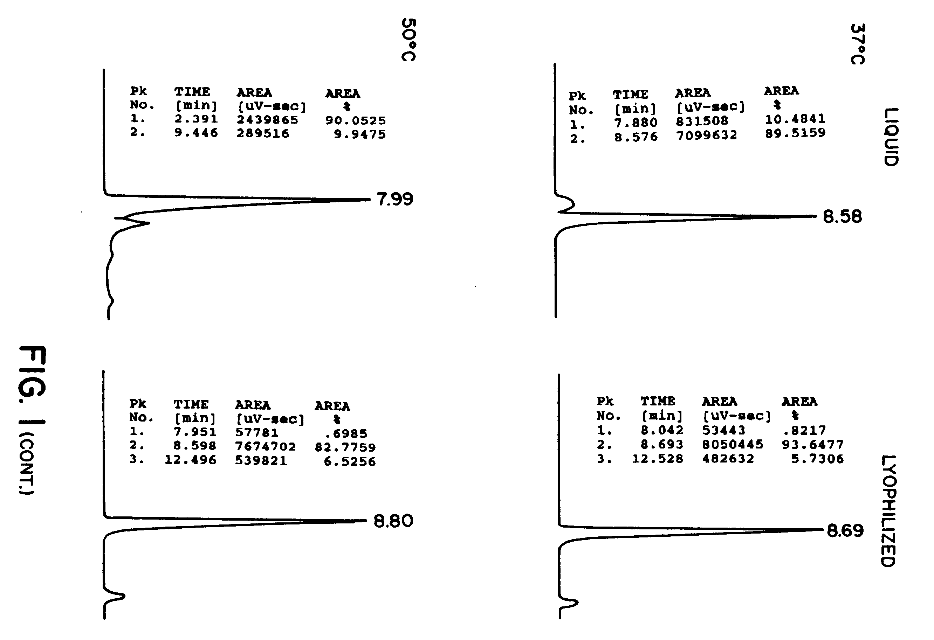

The samples were subjected to HPLC gel filtration chromatography. An HPLC unit (DuPont Zorbarx GF-250 HPLC) was equilibrated with .2 M sodium phosphate buffer (pH 6.8) at a flow rate of 1 ml/min. Absorbance wavelength was set at 214 nm. Undiluted samples were injected and analyzed. The results showed that both liquid and reconstituted samples were relatively stable under the 4°C and 22°C stress conditions. At 37°C and 50°C, the liquid samples showed a measurable increase in dimer concentration generating 10.48% dimer at 37°C and 90.05% dimer at 50°C. The reconstituted samples show no increase in dimer levels at 37°C and 50°C. The results are shown in Figure 1.

### Immunoreactivity Assay

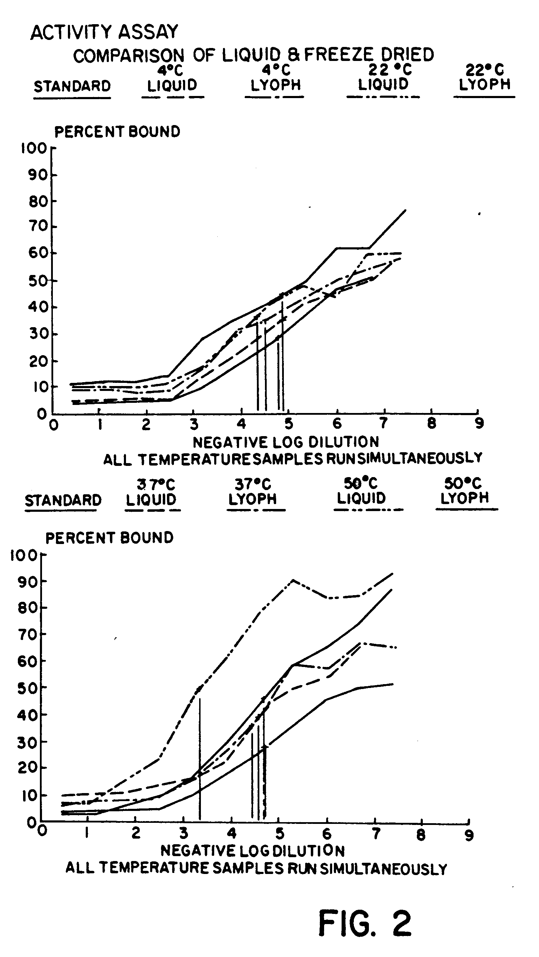

An assay was performed on the samples to determine the relative immunoreactivity of the IgG in the samples. The activity analysis showed that antibody activity was maintained in both liquid and lyophilized samples. The results are shown in Figure 2.

The procedure used for determining antibody immunoreactivity is as follows:

In a polyvinylchloride (PVC) plate labeled "Antigen", 50 µl of diluted antigen was dispensed into each of the 96 wells. The plate was placed uncovered at 37°C to air dry for four hours. After all the solution had evaporated from the wells, each well was washed twice with 100 µl of phosphate buffered saline (PBS). To each well, 200 µl of 5% bovine serum albumin (BSA) in PBS was dispensed, and allowed to incubate covered for 2 hours at room temperature.

In a separate PVC plate labeled "Antibody", 75 ul of 10% BSA in PBS was dispensed into wells in rows 2 through 12. 100 µl of the reconstituted lyophilized samples and standard antibody solutions that had been prediluted to 400 µg/ml were dispensed to wells in row 1. With a pipetting device, 25 µl was removed from row 1, dispensed to row 2 and mixed. This dispensing was repeated for the wells in rows 3 through 11. The final 25 µl increment was discarded. Row 12 was the 100% binding row.

PVC plates labeled "Antigen" were washed twice with 200 µl of PBS. Using new pipette tips each time, 50µl of the prediluted antibody was transferred from the PVC plate labeled "Antibody" to corresponding wells on the "Antigen" plate. To each well on the "Antigen" plate containing 50 µl of prediluted antibody, 20 ng of $^{125}$I labeled antibody in 50 µl of 1% BSA in PBS was added. The plate was covered and incubated at 4°C for 20 hours. After 20 hours at 4°C, the excess radiolabel was washed from the plate with three washes of PBS, and counted in a gamma counter.

The data were analyzed by averaging duplicate counts. Counts per minute (cpm) versus the negative log of the cold antibody were plotted. From row 12 of each antibody dilution series, the number of cpm found, which represents 100% of binding, was calculated. This number was divided by two to obtain the 50% maximal binding value. The 50% maximal binding value for each antibody dilution series was compared to the point on the curve for each antibody titration representing the cpm of the 50% maximal point. The protein concentration was determined from this point.

### SDS:PAGE Chromatography

The samples were subjected to SDS:PAGE chromatography to determine molecular integrity of the IgG after stressing at various temperatures. Non-reduced samples were prepared in a microfuge tube. Protein sample, PBS, and SDS and coomassie stock were mixed to obtain a protein concentration of 2mg/ml. Fifty microliters (50 $\mu$l) of the protein solution was reduced by adding 5 $\mu$l of $\beta$-mercapto-ethanol. The samples in tubes were exposed to boiling water for 2 minutes. Using a 1 ul applicator comb, each prepared sample was applied to the gel (Pharmacia Phast Gel SDS Page Gels (8-25%)). The gel was run according to the manufacturers instructions.

SDS:PAGE chromatography demonstrated the molecular integrity of the lyophilization material throughout the range of stress temperatures. The liquid formulation maintained its structure at 4°C and 22°C, but begins to show increasing fragmentation at 37°C and 50°C.

### Native PAGE Chromatography

The samples were tested using Native PAGE chromatography. The samples were diluted with PBS (phosphate buffered saline), and applied to Native Page Gel (Pharmacia Phast Gel Native Page Gel 7.5% and 12.5% homogeneous). The gels were run, stained and preserved according to the manufacturer's instructions. Native PAGE results showed some alterations in the proteins exposed to different temperatures, for both reconstituted lyophilized samples and liquid samples. The proteins in the liquid samples become more negatively charged at higher temperatures. Increases in negativity were observable with liquid samples stored at 22°C and 37°C, with no protein entering the gel at 50°C indicating extensive protein denaturation. The reconstituted lyophilized samples were observable in the gel throughout the temperature range, and the lyophilized 50°C sample is less negative than the liquid 37°C sample. This phenomenon was consistent in both the 7.5% and 12.5% homogeneous gels, with the 12.5% gel appearing as more well-defined bands.

### Isoelectric Focusing

Isoelectric focusing was conducted to determine any changes in the protein isoelectric point (pI) between the liquid and lyophilized samples. Samples were diluted with PBS and applied to an isoelectric fosucing gel (Pharmacia Phast Gel pH 3-9 Isoelectric Focusing Gel). The gel was run, fixed, stained and destained according to manufacturer's instructions. The results showed no alteration in the pI of the protein at any of the stress temperatures for the lyophilized product. The liquid samples demonstrated no alteration at 4°C and 22°C, but showed considerable movement toward the lower pH range at 37°C, and denaturation at 50°C.

### Residual Moisture Analysis

Residual moisture analysis was performed on placebo lyophilized samples to determine the residual moisture attributable to the solution without protein. Placebo vials contained a solution of 10 mM sodium acetate (pH 4.5), and 10% maltose. The placebo vials were lyophilized along with the vials containing antibody under the same conditions. For the residual moisture analysis, the contents of placebo vials were removed into a dry pre-tared weigh boat (VWR Scientific) in a glove bag which had been previously purged with dry, inert gas. The sample was weighed to obtain an initial weight of the placebo material. The weigh boat with the placebo material was then placed in a vacuum oven (VWR Scientific) at 60°C, and pressure in the vacuum oven was reduced for 24 hours. The weigh boats were then removed from the oven into a glove bag purged with dry nitrogen gas, and allowed to equilibrate to room temperature. The samples were then reweighed. The sequence was repeated until the samples were brought to constant weight (there were no more reductions in weight(. The final constant weight was divided by the original weight to calculate the precent residual moisture. The results are shown in Table 4.

## Table 4
### Residual Moisture Analysis (lyophilized)

| Vial# | Weigh Boat Tare (g) | Weigh Boat + Placebo | Placebo | Placebo After Drying | Loss In Drying | % Residual Moisture |
|---|---|---|---|---|---|---|
| 1 | 1.57387 | 2.41015 | 0.83628 | 0.81698 | 0.01930 | 2.31 |
| 2 | 1.54695 | 2.42704 | 0.88009 | 0.86034 | 0.01975 | 2.24 |
| 3 | 1.58552 | 2.44362 | 0.85810 | 0.83867 | 0.01943 | 2.26 |

AVERAGE VALUE = 2.27%

Example 2. Preparation of a Lower Maltose Lyophilized Formulation for IgG

Solutions of immunoglobulin G were prepared and tested according to the procedures set out in Example 1, but reducing the amount of maltose in the formulation from 10% to 2%. This formulation consisted of 10mM sodium acetate (pH 4.5), 2% w/v maltose and IgG.

Lyophilization

Twenty milliliter vials were prepared and filled with 10 ml of the reduced-maltose formulation and placed in the freeze dryer. Placebo vials, filled with the identical solution of buffer and maltose, but lacking antibody, were also placed in the freeze dryer. Lyophilization was carried out as described in Example 1.

Temperature stress testing of the lyophilized product and the placebo vials was done by storing the test samples at 4, 22 and 40°C for 52 days. Some of the lyophilized samples were then reconstituted with 10 ml of water. The reconstitution times are shown in Table 5.

## Table 5

### Reconstitution Times (Lyophilized Product)

| Sample | Reconstituion Time (Minutes) |
|---|---|
| $t_o$ | 0.5 |
| 52 day samples | |
| 4°C | 0.67 |
| 22°C | 0.75 |
| 40°C | 0.83 |

Visual examination of the vials for particulates was then performed. Visual examination showed no visible particulates in the reconstituted lyophilized samples. The results are shown in Table 6.

## Table 6

### Visual Examination

| T°C | Formulation | Observation |
|---|---|---|
| 4 | lyophilized | Clear |
| 22 | lyophilized | Clear |
| 40 | lyophilized | Clear |

### Liquidborne Particle Analysis

Liquidborne particle analysis showed that all samples were within USP specifications for 10 and 25 micron-sized particles. The results are shown in Table 7.

## Table 7

### Subvisible Liquidborne Particle Analysis (52 Days)

| T°C | DIFFERENTIAL | | | | | | TOTAL | | | | | |
|---|---|---|---|---|---|---|---|---|---|---|---|---|
| | 5μ | 10μ | 20μ | 25μ | 30μ | 50μ | 5μ | 10μ | 20μ | 25μ | 30μ | 50μ |
| 4 | 313 | 54 | 2 | 2 | 0 | 0 | 376 | 58 | 4 | 2 | 0 | 0 |
| 22 | 910 | 142 | 10 | 2 | 4 | 0 | 1068 | 158 | 16 | 6 | 4 | 0 |
| 40 | 676 | 108 | 4 | 2 | 2 | 0 | 794 | 118 | 10 | 6 | 4 | 0 |

### HPLC Gel Filtration Chromatography

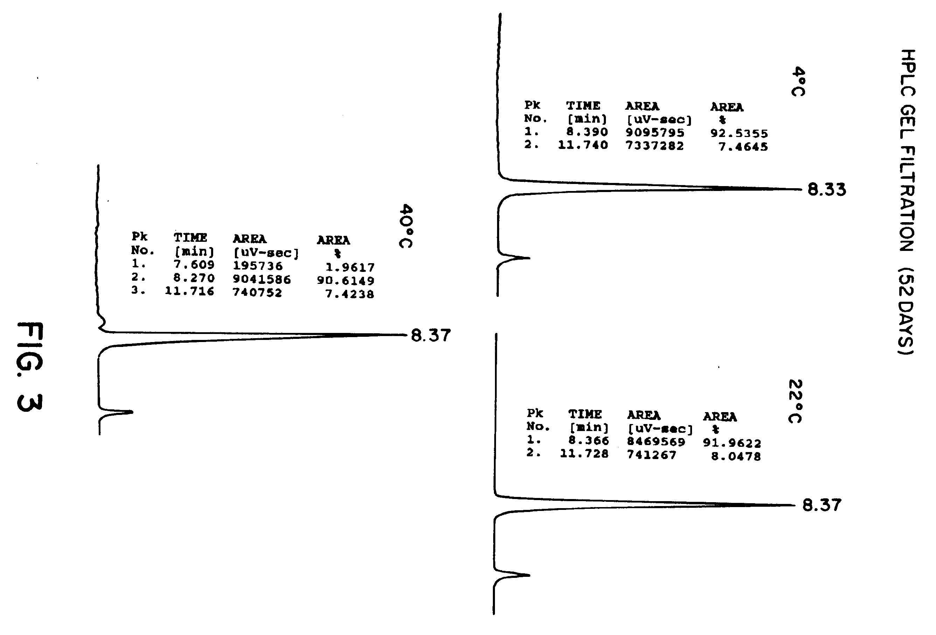

Gel filtration results showed that the reconstituted material stressed at 4°C and 22°C were identical with the starting material. At 40°C, however, some dimer formation was apparent (about 2.12%). The results are shown in Figure 3.

### Immunoreactivity Assay

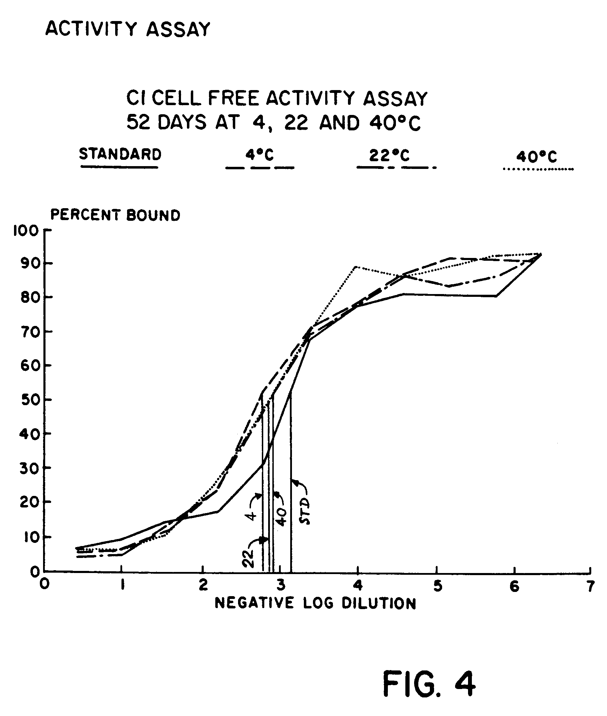

Immunoreactivity assay results indicate that all samples performed within specification for the assay, and possessed activity comparable to the standard. The results are shown in Figure 4.

### SDS:PAGE Chromatography

SDS-PAGE gels for all samples appeared the same when loaded at 2 mg/ml on the non-reduced and reduced gels. When samples were loaded at 1 mg/ml, some aggregated material was observed only in the 40°C sample.

### Native Page Chromatography

Native:PAGE gels (both 7.5% and 12.5% homogenous) exhibited a slight alteration in surface charge in the 40°C stressed samples.

### Isoelectric Focusing

Isoelectric focusing of the freeze dried samples showed no change in the banding patterns at any of the temperature conditions.

Residual Moisture

Residual moisture analysis performed on three of the placebo vials showed an average residual moisture value of 1.24%. The results are shown in Table 8.

## Table 8

### RESIDUAL MOISTURE ANALYSIS

| VIAL # | WEIGH BOAT TARE (g) | WEIGH BOAT +PLACEBO | PLACEBO | PLACEBO AFTER DRYING | LOSS IN DRYING | % RESIDUAL MOISTURE |
|--------|---------------------|---------------------|---------|---------------------|----------------|---------------------|
| 1 | 1.58839 | 1.77753 | 0.18914 | 0.18555 | 0.00359 | 1.90 |
| 2 | 1.54959 | 1.74645 | 0.19686 | 0.19471 | 0.00215 | 1.09 |
| 3 | 1.55476 | 1.71318 | 0.15482 | 0.15726 | 0.00116 | 0.73 |

AVERAGE VALUE = 1.24%

## Claims

1. A monoclonal antibody composition for parenteral administration of Immunoglobulin G comprising a lyophilized formulation of:
   (a) sodium acetate having a pH of between 3 and 6;
   (b) maltose; and
   (c) monoclonal immunoglobulin G or a fragment thereof.

2. A composition of Claim 1 wherein the concentration of sodium acetate is between 5 to 10 mM.

3. A composition of Claim 1 wherein the amount of monoclonal immunoglobulin G is between 1 and 25 mg/ml.

4. A composition of Claim 1 wherein the monoclonal immunoglobulin G comprises immunoglobulin $G_{2a}$.

5. A composition of Claim 1 wherein the monoclonal immunoglobulin G antibody or fragment is radio-labeled.

6. A composition of Claim 1 wherein the amount of maltose is between 2 to 10 percent by weight.

7. A monoclonal immunoglobulin G composition of Claim 1 containing sodium acetate, maltose and immunoglobulin G, wherein the improvement comprises lyophilizing the composition to form a lyophilized cake which may be reconstituted to yield an injectable solution of immunoglobulin G.

8. A method of preparing a monoclonal immunoglobulin G composition of Claim 1 which comprises lyophilizing an immunoglobulin G formulation containing monoclonal immunoglobulin G, sodium acetate and maltose under conditions appropriate to form a lyophilized cake which is capable of being reconstituted into an injectable solution.

9. A method of Claim 8 wherein the composition comprises:
   (a) 5-10 mM sodium acetate having a pH of between about 3-6;
   (b) 2-10 percent by weight maltose;
   (c) 1-25 mg/ml monoclonal immunoglobulin G.

10. A method of Claim 8 wherein the lyophilization conditions comprise:
    a primary drying phase wherein the temperature is between -40°C to -42°C and the pressure is between 6.67 to 10.7 Pa (50 - 80 mTorr); and a secondary drying phase wherein the temperature is ambient and the pressure is between 6.67 to 10.7 Pa (50 - 80 mTorr).

11. A lyophilized cake made by the method of Claim 8 comprising a stable composition of monoclonal immunoglobulin G wherein the cake can be reconstituted to yield a monoclonal immunoglobulin G solution which has a particle count of less that 150 particles of 10 µm size or greater per milliliter, and less than 15 particles of 20 µm, size or greater per milliliter.

12. A lyophilized cake of Claim 11 wherein the cake is reconstituted with water or saline solution.

**Patentansprüche**

1. Eine monoklonale Antikörperzusammensetzung für parenterale Verabreichung von Immunoglobulin G, umfassend eine gefriergetrocknete Formulierung von:
   (a) Natriumacetat mit einem pH zwischen 3 und 6;
   (b) Maltose; und
   (c) monoklonales Immunoglobulin G oder ein Fragment desdelben.

2. Eine Zusammensetzung nach Anspruch 1, bei der die Konzentration von Natriumacetat zwischen 5 bis 10 mM beträgt.

3. Eine Zusammensetzung nach Anspruch 1, bei der die Menge des monoklonalen Immunoglobulins G zwischen 1 und 25 mg/ml beträgt.

4. Eine Zusammensetzung nach Anspruch 1, bei der das monoklonale Immunoglobulin G Immunoglobulin $G_{2a}$ umfaßt.

5. Eine Zusammensetzung nach Anspruch 1, bei der der monoklonale Immunoglobulin G-Antikörper oder sein Fragment radioaktiv markiert ist.

6. Eine Zusammensetzung nach Anspruch 1, bei der die Menge an Maltose zwischen 2 bis 10 Gewichts-% liegt.

7. Eine monoklonale Immunoglobulin G-Zusammensetzung nach Anspruch 1, die Natriumacetat, Maltose und Immunoglobulin G enthält, bei der die Verbesserung umfaßt, daß die Zusammensetzung gefriergetrocknet ist, um einen gefriergetrockneten Kuchen zu bilden, der zur Infusion hergerichtet werden kann, um eine injizierbare Lösung von Immunoglobulin G zu liefern.

8. Ein Verfahren zum Herstellen einer monoklonalen Immunoglobulin G-Zusammensetzung nach Anspruch 1, das das Gefriertrocknen einer Immunoglobulin G-Formulierung, die monoklonales Immunoglobulin G, Natriumacetat und Maltose enthält, unter Bedingungen umfaßt, die geeignet sind, um einen gefriergetrockneten Kuchen zu bilden, der zu einer injizierbaren Lösung für Infusion hergerichtet werden kann.

9. Ein Verfahren nach Anspruch 8, bei dem die Zusammensetzung:
   (a) 5-10 mM Natriumacetat mit einem pH zwischen etwa 3-6;
   (b) 2-10 Gewichts-% Maltose;
   (c) 1-25 mg/ml monoklonales Immunoglobulin G umfaßt.

10. Ein Verfahren nach Anspruch 8, bei dem die Gefriertrocknungsbedingungen:
    eine erste Trocknungsphase, in der die Temperatur zwischen -40° C bis -42° C ist und der Druck zwischen 6,67 bis 10,7 Pa (50 - 80 mTorr) liegt, und eine zweite Trocknungsphase, in der die Temperatur Umgebungstemperatur ist und der Druck zwischen 6,67 bis 10,7 Pa (50 - 80 mTorr) liegt, umfassen.

11. Ein gefriergetrockneter Kuchen, der nach dem Verfahren gemäß Anspruch 8 hergestellt worden ist, der eine stabile Zusammensetzung von monoklonalem Immunoglobulin G umfaßt, wobei der Kuchen für Infusion hergerichtet werden kann, um eine monoklonale Immunoglobulin G-Lösung zu liefern, die eine Teilchenzahl von weniger als 150 Teilchen mit 10 µm Größe oder größer pro Milliliter und weniger als 15 Teilchen mit 20 µm Größe oder größer pro Milliliter aufweist.

12. Ein gefriergetrockneter Kuchen nach Anspruch 11, bei dem der Kuchen mit Wasser oder einer Kochsalzlösung zur Infusion hergerichtet wird.

**Revendications**

1. Composition d'anticorps monoclonal pour l'administration parentérale d'immunoglobuline G, comprenant une préparation lyophilisée
   (a) d'acétate de sodium ayant un pH de 3 à 6;
   (b) de maltose; et
   (c) d'immunoglobuline G monoclonale ou d'un fragment de celle-ci.

2. Composition selon la revendication 1, dans laquelle la concentration d'acétate de sodium est comprise entre 5 et 10 mM.

3. Composition selon la revendication 1, dans laquelle la quantité d'immunoglobuline G monoclonale est comprise entre 1 et 25 mg/ml.

4. Composition selon la revendication 1, dans laquelle l'immunoglobuline G monoclonale comprend de l'immunoglobuline $G_{2a}$.

5. Composition selon la revendication 1, dans laquelle l'anticorps immunoglobuline G monoclonal ou son fragment est marqué radioactivement.

6. Composition selon la revendication 1, dans laquelle la quantité de maltose est comprise entre 2 et 10% en poids.

7. Composition d'immunoglobuline G monoclonale selon la revendication 1, contenant de l'acétate de sodium, du maltose et de l'immunoglobuline G, dans laquelle l'amélioration consiste a lyophiliser la composition pour former un gâteau lyophilisé qui peut être reconstitué pour donner une solution injectable d'immunoglobuline G.

8. Procédé de préparation d'une composition d'immunoglobuline G monoclonale selon la revendication 1, comprenant l'opération consistant à lyophiliser une poréparation d'immunoglobuline G contenant de l'immunoglobuline G monoclonale, de l'acétate de sodium et du maltose, dans des conditions appropriées pour former un gâteau lyophilisé qui est susceptible d'être reconstitué en une solution injectable.

9. Procédé selon la revendication 8, dans lequel la composition comprend:
   (a) 5-10 mM d'acétate de sodium ayant un pH d'environ 3 à 6;
   (b) 2-10% en poids de maltose;
   (c) 1-25 mg/ml d'immunoglobuline G monoclonale.

10. Procédé selon la revendication 8, dans lequel les conditions de lyophilisation comprennent une phase de séchage primaire dans laquelle la température est comprise entre -42°C et -40°C et la pression est comprise entre 6,67 et 10,7 Pa (50-80 mtorr), et une phase de séchage secondaire dans laquelle la température est la température ambiante et la pression est comprise entre 6,67 et 10,7 Pa (50-80 mtorr).

11. Gâteau lyophilisé obtenu par le procédé selon la revendication 8, comprenant une composition stable d'immunoglobuline G monoclonale, lequel gâteau peut être reconstitué pour donner une solution d'immunoglobuline G monoclonale qui a une teneur en particules de moins de 150 particules de 10 μm de grosseur ou moins par millilitre et de moins de 15 particules de 20 μm de grosseur ou plus par millilitre.

12. Gâteau lyophilisé selon la revendication 11, lequel gâteau est reconstitué avec de l'eau ou une solution saline.

HPLC GEL FILTRATION

FIG. I

LIQUID

LYOPHILIZED

37°C

8.58

8.69

50°C

7.99

8.80

FIG. I (CONT.)

FIG. 2

## HPLC GEL FILTRATION (52 DAYS)

**4°C** — 8.33

| Pk No. | TIME [min] | AREA [uV-sec] | AREA % |
|---|---|---|---|
| 1. | 8.390 | 9095795 | 92.5355 |
| 2. | 11.740 | 7337282 | 7.4645 |

**22°C** — 8.37

| Pk No. | TIME [min] | AREA [uV-sec] | AREA % |
|---|---|---|---|
| 1. | 8.366 | 8469569 | 91.9622 |
| 2. | 11.728 | 741267 | 8.0478 |

**40°C** — 8.37

| Pk No. | TIME [min] | AREA [uV-sec] | AREA % |
|---|---|---|---|
| 1. | 7.609 | 195736 | 1.9617 |
| 2. | 8.270 | 9041586 | 90.6149 |
| 3. | 11.716 | 740752 | 7.4238 |

## FIG. 3

ACTIVITY ASSAY

CI CELL FREE ACTIVITY ASSAY
52 DAYS AT 4, 22 AND 40°C

STANDARD          4°C          22°C          40°C

FIG. 4